# EUROPEAN PATENT SPECIFICATION

(11) **EP 2 205 551 B1**
(45) Date of publication and mention of the grant of the patent: **08.01.2014**
(21) Application number: 08746586.0
(22) Date of filing: 23.04.2008
(51) Int. Cl.: C07C 213/08, C07C 217/40

(54) **METHOD OF PRODUCING AMINO DIALKOXY ALDEHYDES VIA HYDROFORMYLATION**
VERFAHREN ZUR HERSTELLUNG VON AMINODIALKOXYALDEHYDEN DURCH HYDROFORMYLIERUNG
PROCÉDÉ DE PRODUCTION D'ALDEHYDES AMINO DIALCOXYLÉS PAR LE BIAIS DE L'HYDROFORMYLATION

(30) Priority: 26.04.2007 US 926386 P
(43) Date of publication of application: 14.07.2010
(73) Proprietor: Dr. Reddy's Laboratories (EU) Limited, Beverley East Yorkshire HU17 0LD (GB)
(72) Inventor: COBLEY, Christopher, Cambs CB4 9JU (GB); RAND, Cynthia, L., Sanford, MI 48657 (US)
(74) Representative: Raynor, John
(86) International application number: PCT/US2008/061194
(87) International publication number: WO 2008/134327

(56) References cited:
- BALENOVIC, BREGANT, GALIJAN, STEFANAC, SKARIC: "Optically active amino aldehydes. II. Preparation of cyclic acetals of quaternary amino aldehydes; contribution to the knowledge of the stereospecificity of muscarinic activity" JOURNAL OF ORGANIC CHEMISTRY, vol. 21, 1956, pages 115-118, XP002493039
- DELOGU, FAEDDA, GLADIALI: "Hydrocarbonylation of unsaturated nitrogen compounds. Synthesis of N-protected aminoacid derivatives from N-substituted phthalimides" JOURNAL OF ORGANOMETALLIC CHEMISTRY, vol. 268, 1984, pages 167-174, XP002493040
- GRIBBLE, SWITZER, SOLL: "A biomimetic approach to the Elaocarpus alcaloids. Syntheses of (+/-)-elaeokanine C, (+/-)-elaeocarpidine and (+/-)-tarennine" JOURNAL OF ORGANIC CHEMISTRY, vol. 53, 1988, pages 3154-3170, XP002493041

## Description

### Field of the Invention

The present invention is a method of producing ami no dialkoxy compounds, including particularly 4-aminobutyraldehyde dialkyl acetal.

### Background of the Invention

Amino dialkoxy compounds have utility as bifunctional synthetic intermediates for use in pharmaceutical, agrochemical, fragrance chemical and other fine chemical applications. Compositions having a low purity (about 90%) 4-aminobutyraldehyde diethyl acetal are available commercially for example from Sigma-Aldrich. Most previous methods of making this compound involve reduction of a nitrile functionality to access the primary amine - a step that is either susceptible to catalyst poisoning via trace quantities of contaminants or involves stoichiometric hydride reducing agents. See e.g. U.S. 5,886,227. The present inventors are unaware of any methods to make such material efficiently in high purity and relatively large scale production.

Balenovic et al, Journal of Organic Chemistry, vol. 21, 1956, p115-118 describes preparation of cyclic amino acetals from the corresponding amino acids, or from the well crystallized tetra-hydroimidazole derivatives of the phthalimidoaldehydes.

Delogu et al, Journal of Organometallic Chemistry, vol. 268, 1984, p167-174 describes hydrocarbonylation of some beta-unsaturated N-vinylphthalimides catalysed by Rh or Pd complexes.

Gribble et al, Journal of Organic Chemistry, vol. 53, 1988, p3164-3170 describes a biomimetic approach syntheses of (+/-)-elaeokanine A, (+/-)-elaeokanine C, (+/-)-elaeocarpidine, and (+/-)-tarennine.

### Summary of the Invention

The method of the present invention provides an improved method for making amino dialkoxy compounds. This method comprises:
(a) providing an N-alkenylphthalimide;
(b) converting the alkenyl group to an aldehyde by hydroformylation;
(c) reacting the aldehyde with an alcohol or alkyl diol to form two alkoxy groups on or a cyclic diether with the carbon that was part of the aldehyde group - i.e. forming an acetal or cyclic acetal moiety; and
(d) then forming a primary amine by deprotection of the amine (i.e. removal of the phthalimide functionality);
wherein the hydroformylation step occurs in the presence of syngas and a catalyst which is a complex of rhodium with a bidentate phosphorous containing ligand.

### Detailed Description of the Invention

Preferably, the alkenyl group of steps a and b is an alkenyl of up to 5 carbon atoms, more preferably 3 or 4 carbon atoms and most preferably 3 carbon atoms (i.e. an allyl group - also referred to as propenyl group).

The first step of providing the N-alkenylphthalimide may be undertaken by any known method. For example, one may react a potassium phthalimide (or analogous compound) with a alkenyl halide such as alkenyl chloride or alkenyl bromide; one may react an alkenyl amine with a phthalic anhydride; one may use preformed potassium phthalimide or form the potassium phthalimide in situ; or use these methods with or without a quaternary ammomiun salt as catalyst. See e.g. Abulikemu, Abudurexiti; Halasz, Gabor; Csampai, Antal; Gomory, Agnes; Rabai, Jozsef, Improved synthesis of perfluorooctylpropyl amine, Journal of Fluorine Chemistry (2004), 125(7), 1143-1146, describing the synthesis of *N*-allyl phthalimide (75% yield) via the reaction of phthalic anhydride and allyl amine in acetic acid at reflux over two hours; Koehling, Petra; Schmidt, Axel M.; Eilbracht, Peter, Tandem Hydroformylation/Fischer Indole Synthesis: A Novel and Convenient Approach to Indoles from Olefins, Organic Letters (2003), 5(18), 3213-3216, describing the reaction of potassium phthalimide with allyl bromide in dimethylformamide (DMF) over 24h to yield N-allyl phthalimide in 72% yield; Pawar, N. S.; Attarde, S. B.; Dalal, D. S.; Sonar, G. R.; Kapadi, U. R.; Hundiwale, D. G.; Kumbhar, P. P, Synthesis of N-alkyl and N-acyl phthalimides using polymer-supported phthalimide anion, Journal of Indian Council of Chemists (2001), 18(2), 29-31, describing the reaction of phthalimide with allyl bromide in the presence of KOH and Amberlite IRA 400 to yield *N*-allyl phthalimide in 91% yield; Daiss, Juergen O.; Duda-Johner, Seraina; Burschka, Christian; Holzgrabe, Ulrike; Mohr, Klaus; Tacke, Reinhold, N+/Si Replacement as a Tool for Probing the Pharmacophore of Allosteric Modulators of Muscarinic M2 Receptors: Synthesis, Allosteric Potency, and Positive Cooperativity of Silicon-Based W84 Derivatives, Organometallics (2002), 21(5), 803-811, reporting the reaction of potassium phthalimide with allyl chloride to give *N*-allyl phthalimide in 62% yield; Heyes, James A.; Niculescu-Duvaz, Dan; Cooper, Robert G.; Springer, Caroline J. Synthesis of Novel Cationic Lipids: Effect of Structural Modification on the Efficiency of Gene Transfer, Journal of Medicinal Chemistry (2002), 45(1), 99-114, describing the reaction of potassium phthalimide with allyl bromide in DMF to yield N-allyl phthalimide in 80% yield; Khatri, Dilip; Rajora, Sonal; Banu, Tahira; Talesara, G. L. Synthesis of bis-1,2-aryl-biguanidino-oxy, 3-biguanidinopropane as potential antimalarials, Asian Journal of Chemistry (1999), 11(4), 1438-1444, describing the reaction of potassium phthalimide with allyl bromide in the presence of tetrabutylammonium bromide in toluene to yield N-allyl phthalimide in 80% yield; Vlassa, M.; Kezdi, Maria; Fenesan, Maria, Preparation of N-alkylphthalimides using solid-liquid phase transfer catalysis without solvent. Revue Roumaine de Chimie (1989), 34(7), 1607-9, describing the reaction of potassium phthalimide with allyl bromide in the presence of tetrabutylammonium bromide to yield N-allyl phthalimide in 95% yield; and Anelli, Pier Lucio; Montanari, Fernando; Pollak, Vladimir; Quici, Silvio. Synthesis of crown ethers bonded to a polystyrene matrix through an amido linkage and their use as phase-transfer catalysts, Gazzetta Chimica Italiana (1986), 116(3), 127-31, describing the reaction of potassium phthalimide with allyl bromide in the presence of phosphonium salt and toluene to yield N-allyl phthalimide in 92% yield.

According to a particularly preferred embodiment, a potassium phthalimide compound is reacted with an alkenyl chloride in an organic solvent at a temperature of at least room temperature but preferably no more than 100° C, more preferably no more than 60° C. The organic solvent is preferably an aprotic solvent, more preferably is selected from toluene, dimethylformamide (DMF), tetrahydrofuran (THF), acetonitrile, ethylacetate or tert-butylmethylether, and most preferably is DMF. The reactants are preferably present in approximately stoichiometric amounts to each other. An ammonium salt may be added, preferably a quaternary ammonium salt, in order to aid the reaction by increasing conversion and yields. The reactants are preferably present in concentrations of weight reactant to volume solvent percentage ratio (w/v%) between 50 w/v% and 5 w/v%, and more preferably between 50 w/v% and 25 w/v%. The reaction may be quenched by addition of water. The product may be precipitated preferably at reduced temperatures. The precipitate may be washed and recrystallized, for example from a lower alcohol such as ethanol to yield the N-alkenyl phthalimide. The yield of this first step is preferably at least 60%, more preferably 70% or more.

In the second step of the method the *N*-alkenyl phthalimide is hydroformylated to form an aldehyde. The hydroformylation step occurs in the presence of a hydroformylation catalyst and syn gas. The syn gas preferably comprises CO and H₂ in mole ratios of 0.1:1 to 1:0.1, more preferably in mole ratio of 0.9:1 to 1:0.9. Preferred catalysts are those that will provide regiospecificity for the resulting aldehydes (e.g. will provide more of the desired regiospecific aldehyde). Suitable catalysts for this purpose comprise rhodium (Rh) complexed to a bidentate phosphorus-containing ligand.

Preferably, the ligand is a bisphosphite represented by formula (**1**), wherein R¹ is tert-butyl, methoxy or 2,4,6-trimethylphenyl; R² is tert-butyl or trimethylsilyl; A is the structural fragment according to formula **I** in which R³ is tert-butyl, methoxy or hydrogen and R⁴ is tert-butyl, trimethylsilyl or hydrogen; B is the same as A when R³ and R⁴ are both hydrogen or, when R³ and R⁴ are not both hydrogen, a structural fragment according to formulae **II** or **III**, in which R⁵ through R⁸ are alkyl, halogen, methoxy or hydrogen.. Representative ligands of this type, having utility in the process of the invention, are selected from the group including BIPHEPHOS (**2**), (**3**) and the unsymmetrical bisphosphite (**4**) in which R is H, CH₃, OCH₃, or OC₂H₅. A rhodium complex of BIPHEPHOS (**2**) is a particularly preferred catalyst.

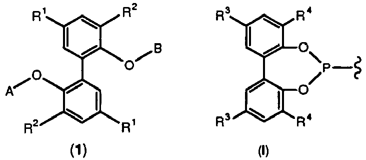

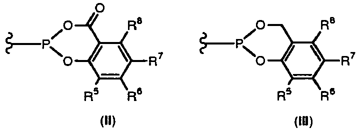

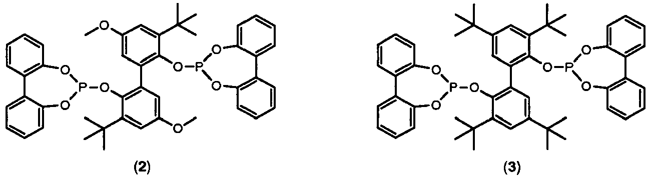

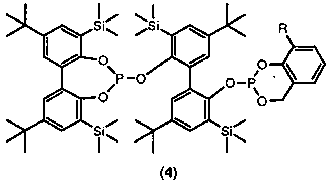

For operation of the process of the invention, a pre-formed, storage-stable complex of the transition metal and phosphorus-containing ligand may be employed, although more commonly, the catalyst complex is prepared in solution prior to use, and said solution is combined in the reaction vessel with a solution of the alkenyl substrate and syngas. Preparation of the solution of catalyst complex entails reaction of the ligand with a precursor complex containing the transition metal, optionally using a molar excess of ligand such that uncomplexed ligand is present once the entire precursor complex is consumed. Preferably the molar ratio of ligand:transition metal is in the range of about 0.75:1 to 100:1, and more preferably this ratio is in the range of about 0.75:1 to 3:1. Where the transition metal is Rh, the precursor complex is preferably a Rh(I) or Rh(III) complex, more preferably selected from Rh₆(CO)₁₆, Rh₄(CO)₁₂, [Rh(COD)₂]BF₄, [RhCl(COD)]₂, [RhCl(COE)₂]₂, Rh₂(acetate)₄, Rh(acac)₃, RhCl₃ and Rh(COD)(acac), and most preferably is Rh(acac)(CO)₂. In the aforementioned complexes the following abbreviations apply: COD= 1,5-cyclooctadiene, COE = cyclooctene, acac = acetylacetonato.

Preferably this reaction occurs at a temperature of at least room temperature, more preferably at least 60 ° C and a temperature of no greater than 150 ° C, more preferably no greater than 100 ° C. The pressure of the reaction is preferably maintained in the range of 10 to 250, more preferably 20-60, pounds per square inch (psi) gauge pressure. The reaction preferably occurs in a suitable organic solvent such as toluene, tetrahydrofuran, or other solvents that are characterized as being aprotic. The concentration of the N-alkenyl phthalimide is preferably between 50 w/v% and 5 w/v%, and more preferably between 50 w/v% and 20 w/v%. The mole ratio of the N-alkenyl phthalimide to catalyst is preferably at least 1000:1, more preferably at least 2500:1.

For the preferred alkenyl group which is allyl, the hydroformylation reaction preferably yields a molar ratio of linear to branched aldehyde of at least 2:1, more preferably at least 5:1, and even more preferably at least 10:1. The yield of the hydroformylation step is preferably at least 90%, more preferably at least 95%.

The hydroformylation of *N*-acylamino olefins to yield *N*-acyl amino aldehydes, in particular the use of N-phthalimido allylamine and N-diacetyl allyl amine has been reported (see N-Acylamino aldehydes Ajinomoto Co. Inc., (1963), FR 1341874 19631102). The catalysts employed were non-ligand modified Rh or Co carbonyl complexes and as a result, the conditions reported were harsh (30 - 150°C, 710 to 14,210 psi, with CO:H₂ ratio being between 1:0.5 and 1:10). The examples given were run at 120°C and 2,349 psi, yielding the corresponding aldehydes in 78% yield and with a linear to branched product ratio of 7:3.

Hydroformylation of the *N*-allyl phthalimide in the presence of a hydrazone has been reported (see, *2,3-Disubstituted indoles from olefins and hydrazines via tandem hydroformylation-Fischer indole synthesis and skeletal rearrangement.* P. Linnepe, A.M. Schmidt and P. Eilbracht, Organic & Biomolecular Chemistry (2006), 4, 302-313. This resulted in a mixture of regioisomers that spontaneously formed the corresponding indoles *in situ.* A Rh-Xantphos system together with a Syngas ratio of 5:2 (CO:H₂) was employed, but linear to branched product ratio for the reaction was not stated.

In step (c), the aldehydes from step (b) may be directly converted to the acetals without purification or separation of the regioisomers. This reaction may occur via any known method of forming acetal from aldehydes, but preferably comprises contacting the aldehyde with an alcohol or diol to form acetal or cyclic acetal functionality. This reaction is preferably run by addition of the alcohol or diol and an acid catalyst according to known methods (for a review see, "Protective Groups in Organic Synthesis", Eds T.W. Greene and P.G.M. Wuts, John Wiley & Sons, Inc., 1991, 178-198). The alcohol may be any alcohol but is preferably a lower alcohol of up to 7 carbon atoms, preferably up to 2 carbon atoms and most preferably is methanol. A diol of 2 to 5 carbon atoms, preferably up to 2 carbon atoms may be used to provide a cyclic acetal group is desired. The amount of acid catalyst used is preferably less than 5 mol% and more preferably less than 1 mol%. This reaction is preferably run at a temperature of at least room temperature, but preferably no more than 70 ° C, more preferably no more than 40 ° C. An excess of the alcohol relative to the total amount of aldehydes may be used and in some cases may constitute the bulk reaction solvent. Yield of acetals is preferably at least 80%, more preferably at least 90%, with a ratio of linear to branched acetals of at least 2:1, more preferably at least 5:1, and even more preferably at least 10:1.

In step (d), the acetal compounds from step (c) are then reacted to remove the phthalimide group. This reaction may occur by use of known deprotecting agents according to standard methods as described in the literature (for a review see, "Protective Groups in Organic Synthesis", Eds T.W. Greene and P.G.M. Wuts, John Wiley & Sons, Inc., 1991, 358-359). MeNH₂ is a preferred deprotecting agent, because the resulting phthalimide adduct may be easily removed by filtration or extraction in an aqueous basic solution. Alternatively, deprotection could occur via use of hydrazine, a strong base, or a less soluble amine that would enable more effective precipitation of the phthalimido amine by-product. The preferred deprotection occurs in an alcohol solvent and MeNH₂ at concentrations of 2 to 10 Molar, preferably 2 to 8 Molar. The concentration of the phthalimide acetal concentration is preferably between 150 w/v% and 10 w/v%, and more preferably between 100 w/v% and 20 w/v%. The deprotection occurs at room temperature or higher, but preferably at a temperature less than 70° C, more preferably less than 40° C. Yield at this step is preferably at least 80%, more preferably at least 90%.

Preferably the mixture of alkoxy amine compounds is distilled to obtain the desired regioisomer. In a preferred embodiment of the invention, to obtain 4-aminobutyraldehyde dimethyl acetal, the temperature for the distillation is preferably 100 to 150° C at a pressure of 15 to 50 millibar.

Thus, according to a preferred embodiment, the reaction proceeds according to the following scheme:

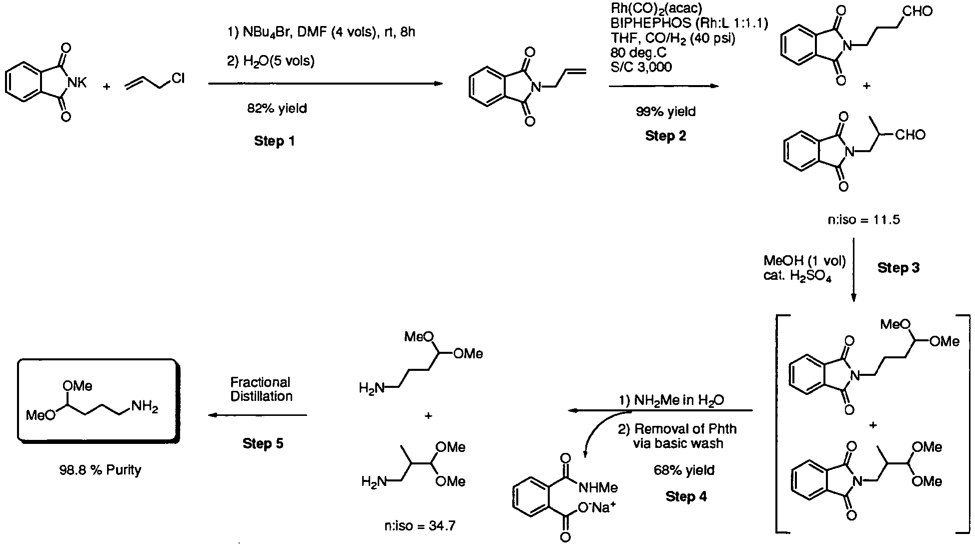

### Examples

### Step (a): N-vinyl phthalimide formation

### Lab-scale:

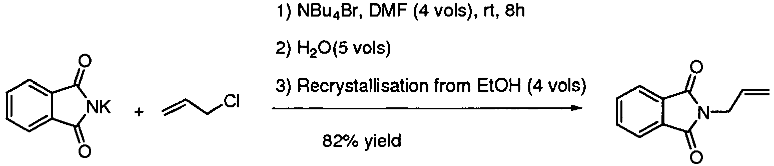

Potassium phthalimide (40.0 g, 0.216 mol) and tetrabutylammonium bromide (1.0 g, 6.204 mmol) were suspended in anhydrous DMF (150 ml). Allyl chloride (17.6 ml, 0.216 mol) was added dropwise over 10 min and the reaction allowed to stir at room temperature for 18 h. The reaction was then quenched with H₂O (200 ml) and the resulting suspension stirred for 10 min (an ice bath was applied during this time to aid precipitation). The resulting precipitate was filtered off, washed with H₂O and dried in air over a slight vacuum for 2 h. The resulting off-white solid was shown by ¹H and ¹³C NMR spectroscopy to be the desired compound (32.8 g, 81.0% yield). This crude material (76.3 g) was subsequently recrystallised from ethanol (270 ml) to yield the desired compound as a pure white solid (67.44g, 88.4% recovery).

¹H NMR (CDCl₃, 400 MHz): δ 4.30 (2H, CH₂, d, *J*³ 5.7 Hz), 5.20 (1H, C*H*H=CH, dd, *J*³ 10.0 Hz, *J*² 1.1 Hz), 5.25 (1H, C*H*H=CH, dd, *J*³ 16.9 Hz, *J*² 1.1 Hz), 5.89 (1H, CHH=C*H*, m), 7.73 (2H, Ar, m), 7.86 (2H, Ar, m).
¹³C{¹H} NMR (CDCl₃, 100 MHz): δ 40.4, 118.1, 123.7, 131.9, 132.5, 134.4, 168.3.

### Larger scale preparation:

| | | |
|---|---|---|
| Quantities : | 950 g | potassium phthalimide |
| | 3.561 | dimethylformamide |
| | 23.8 g | tetrabutylammonium bromide (TBAB) |
| | 391.9 g | allyl chloride |
| | 4.751 | water |
| | 3.01 | water wash |

Potassium phthalimide and TBAB were charged to a 10 1 glass jacketed reactor, DMF was added and the mixture slurried at 15-20 °C. Allyl chloride was ran in over 20 minutes giving no apparent exotherm, then the mixture was stirred overnight at ambient temperature to give a mobile slurry.

Water was added to the reaction mixture over 1 hour giving a slight exotherm and a white slurry, the temperature was maintained below 32 °C using 10 °C glycol in the reactor jacket. The mixture was filtered on a Buchner funnel under vacuum and washed thoroughly with water to give 1154.3 g of damp product. This was dried overnight at 45 °C in vacuo giving 771.0 g white solid product.

### Step (b): Hydroformylation of N-allylphthalimide:

### Lab-scale:

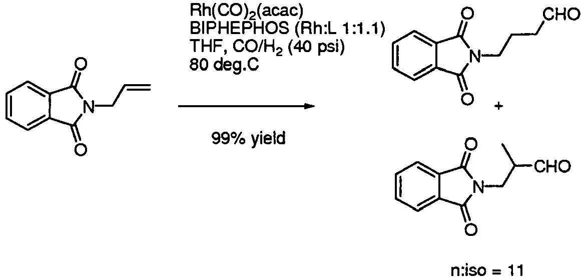

The reaction was performed in a 600 ml Parr pressure vessel fitted with a glass liner, double impeller, injection port, bursting disk, pressure relief valve and pressure gauge. The glass liner was charged with Rh(CO)₂(acac) (18.4 mg, 0.07 mmol) and BIPHEPHOS (58.8 mg, 0.075 mmol), the vessel sealed and flushed five times with nitrogen (145 psi). THF (40 ml, anhydrous and deoxygenated) was added to the vessel via the injection port and the system flushed three times with Syn gas (145 psi, 1:1). The vessel was heated to 40 °C, charged to 80 psi with Syn gas (1:1) and stirred (1,000 rpm) for 20 mins. After this time, the stirring was stopped and the pressure released. A THF solution (200 ml) of N-allyl phthalimide (40.0 g, 0.214 mol) was then added via syringe and the vessel flushed a further three times with Syn gas (145 psi, 1:1). The reaction was heated to 80 °C and charged to 40 psi with Syn gas (1:1). The pressure was monitored and maintained at this pressure for 4 h, consumption was seen to stop within 3 h. The stirring was reduced to 500 rpm and the vessel allowed to cool to room temperature, vented to atmosphere, flushed once with nitrogen (145 psi) and opened. An aliquot of the reaction mixture was analysed by LC and ¹H NMR spectroscopy. This showed the reaction had gone to completion with an n:iso ratio of 11.5. The reaction mixture was reduced to dryness to yield a crude cream coloured solid (48.2 g, 103.9%).

### Linear aldehyde

¹H NMR (CDCl₃, 400 MHz): δ 2.02 (2H, CH₂, m), 2.55 (2H, C*H*₂CHO, t, *J*³ 7.4 Hz), 3.74 (2H, C*H*₂N, t, *J*³ 6.9 Hz), 7.73 (2H, Ar, m), 7.85 (2H, Ar, m), 9.78 (1H, CHO, s).
¹³C{¹H} NMR (CDCl₃, 100 MHz): δ 21.6, 37.5, 41.5, 123.7, 132.4, 134.4, 168.8, 201.3.

### Branched aldehyde

¹H NMR (CDCl₃, 400 MHz): δ 1.18 (3H, CH₃, d, *J*³ 7.0 Hz), 2.90 (1H, C*H*CHO, m), 3.82 (1H, C*H*HN, dd, *J*³ 6.6 Hz, *J*² 14.1 Hz), 4.04 (1H, C*H*HN, dd, *J*³ 7.0 Hz, *J*² 14.1 Hz), 7.74 (2H, Ar, m), 7.85 (2H, Ar, m), 9.77 (1H, CHO, d, *J*³ 1.9 Hz).
¹³C{¹H} NMR (CDCl₃, 100 MHz): δ 12.0, 38.5, 46.2, 123.8, 132.2, 134.5, 168.6, 202.6.

LC conditions: Kromasil C₁₈ column, 250x4.6 mm x 5 µm, MeCN/H₂O (45 :55), 230nm, 11 min (N-allyl amine), 7 min (linear aldehyde), 8 min (branched aldehyde).

### Larger scale preparation:

| | | |
|---|---|---|
| Typical quantities : | 200g | *N*-allyl phthalimide |
| | 1.01 | toluene |
| | 91.9 mg | Rh(CO)₂(acac) |
| | 308.2 mg | BIPHEPHOS |
| | 10 mls | toluene |
| | qs | Syn-gas 50/50 |

*N*-allyl phthalimide was charged to a glass liner of a 2 1 Parr vessel, toluene was added and the vessel assembled. This was purged 5 times by pressurising to 6 bar with nitrogen and venting, then purged once with Syn-gas to 40 psi and vented.

Rh(CO)₂(acac) and BIPHEPHOS charged to a glass liner of a 50 ml Parr vessel and the vessel was assembled. This was purged 6 times by pressurising to 4 bar with Syn-gas and venting, degassed toluene was then added through a septum and the vessel charged to 50 psi with Syn-gas. This was then heated to 45 °C for 45 minutes with rapid stirring, then the activated catalyst solution removed into a syringe and transferred to the 21 Parr vessel.

The 2 1 Parr vessel was then charged to 40 psi with Syn-gas and heated to 80 °C, maintaining the desired pressure with constant addition of Syn-gas until gas uptake ceased. The vessel was cooled to 20 °C, purged with nitrogen and sampled. This showed the reaction to be complete (< 1% starting material). The solution was then concentrated on a rotary evaporator at 45 °C to give approximately 250g of off-white solid product containing some residual toluene.

The above procedure was carried out four times using 3 x 200g and 1 x 150g starting material, yielding 942.6g of crude product with an estimated active content of 870g.

### Step (c): Acetal formation

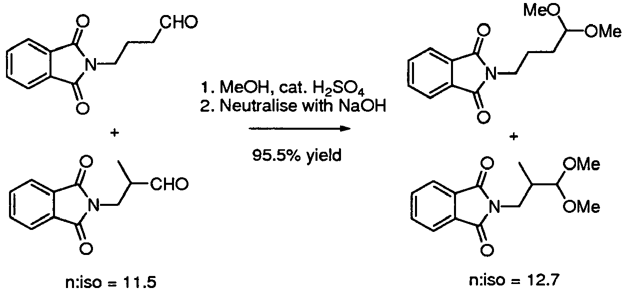

The crude mixture of phthalimido aldehydes produced as detailed above (0.214 mol) was dissolved in MeOH (150 ml) and conc. H₂SO₄ (0.092 g) was added. The reaction was stirred overnight and then neutralized with 4.0M NaOH. All volatiles were removed in vacuo to yield a crude orange oil, which LC and ¹H NMR showed to be the desired dimethyl acetal as a mixture of regioisomers (53.8 g, 95.5% yield, n:iso 12.7).

### Linear dimethylacetal

¹H NMR (CDCl₃, 400 MHz): δ 1.66 (2H, CH₂, m), 1.75 (2H, CH₂, m), 3.31 (6H, CH₃, s), 3.74 (2H, C*H*₂N, t, *J*³ 7.2 Hz), 4.40 (1H, C*H*(OCH₃)₂, t, *J*³ 5.5 Hz), 7.71 (2H, Ar, m), 7.84 (2H, Ar, m).
¹³C{¹H} NMR (CDCl₃, 100 MHz): δ 24.2, 30.2, 38.0, 53.2, 104.4, 123.5, 132.4, 134.2, 168.7.

LC conditions: Kromasil C₁₈ column, 250x4.6 mm x 5 µm, MeCN/H₂O (45 :55), 230nm, 7 min (linear aldehyde), 10 min (linear acetal).

### Steps (c) & (d) combined: Acetal formation and phthalimide removal

### Lab-scale:

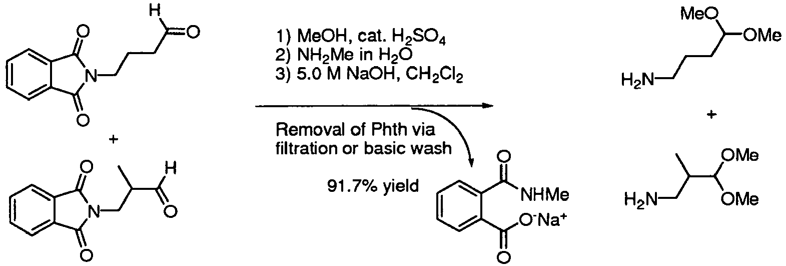

The crude mixture of phthalimido aldehydes (12.57 g, 0.058 mol) produced as detailed above (n:iso 11.3) was suspended in MeOH (12.7 ml) and conc. H₂SO₄ (0.1 ml) was added, resulting in instant dissolution of all solids The reaction was stirred for 1 h and then an aqueous solution of MeNH₂ (40% w/w, 44.9 g 0.58 mol)was added. The reaction was stirred at room temperature overnight during which time a fine white precipitate formed. 5.0 M NaOH(_{aq}) solution (12.73 ml) was added to the main reaction mixture, dissolving the solid, and stirred at room temperature for 2 h. This phase was then extracted twice with MTBE (1 x 20 ml and 1 x 40 ml) and the organic phases separated and combined. Upon removal of all volatiles in vacuo, the residual oil was found to represent only 26% of the expected mass recovery. NaCl (5 g) was added to the aqueous phase, which was then extracted with dichloromethane (40 ml). Removal of all volatiles yielded a clear colourless oil that represented 70% of the expected mass recovery. GC, ¹H and ¹³C NMR showed the combined oils to be a mixture of regioisomers of the free aminobutyraldehyde dimethyl acetals (n:iso 28.4) together with small amounts of H₂O, MeOH and toluene (7.41 g, 96%).

### 4-Aminobutyraldehyde dimethyl acetal

¹H NMR (CDCl₃, 400 MHz): δ 1.51 (2H, CH₂, m), 1.63 (2H, CH₂, m), 1.9 (2H, NH₂, bs), 2.71 (2H, C*H*₂N, t, *J*³ 7.1 Hz), 3.33 (6H, CH₃, s), 4.38 (1H, C*H*(OCH₃)₂, t, *J*³ 5.5 Hz).
¹³C{¹H} NMR (CDCl₃, 100 MHz): δ 28.9, 30.2, 42.1, 53.1, 104.8.

### 3-Amino-2-methylproprylaldehyde dimethyl acetal

¹H NMR (CDCl₃, 400 MHz); δ 0.94 (3H, CH₃, d, ³*J* 6.6 Hz), 1.16 (1H, NH₂, bs), 1.83 (1H, CH, m), 2.57 (1H, C*H*₂N, m), 2.79 (1H, C*H*₂N, m), 3.36 (3H, CH₃, s), 3.38 (3H, CH₃, s), 4.15 (1H, C*H*(OCH₃)₂, d, ³*J* 6.6 Hz).
¹³C{¹H} NMR (CDCl₃, 100 MHz): δ 13.4, 37.0, 39.1, 53.9, 54.9, 108.4.

GC Conditions: ZB5 column, 30 m x 0.32 mm x 1.0 µm, MeCN for sample preparation, 160 °C for 6 min, 4 min (branched dimethyl acetal), 5 min (linear dimethyl acetal).

### Larger scale preparation:

| | | |
|---|---|---|
| Quantities: | 906.7 g | Stage 2 crude product (estimated 825g pure) |
| | 660 g | methanol |
| | 0.5 g | sulphuric acid 98% |
| | 2.95 kg | methylamine 40% w/w aqueous |
| | 522 g | sodium hydroxide 32% |
| | 400 g | sodium chloride |
| | 3.251 | dichloromethane (DCM) |

The crude concentrated hydroformylation product was charged to a 3 1 flask with methanol, sulphuric acid added and the reaction stirred at ambient temperature to give a clear pale yellow solution. This was then transferred to a 10 1 glass jacketed reactor and methylamine solution added, giving an instant exotherm to 35 °C. The reaction was cooled to ambient temperature and stirred for 24 hours, with a thick white precipitate forming after approximately 2 hours.

Sodium hydroxide solution was ran in over 1 hour to give a cloudy solution, which was stirred for 2 hours at ambient temperature. Sodium chloride was added and the reaction stirred to aid dissolution, then multiple phase separations were carried out using a total of 3.25 litres of dichloromethane in quantities between 0.25 1 and 0.75 1. Combined DCM extracts were then concentrated on a rotary evaporator to give 345.0 g of product as a pale yellow liquid.

### Step (e): Fractional distillation

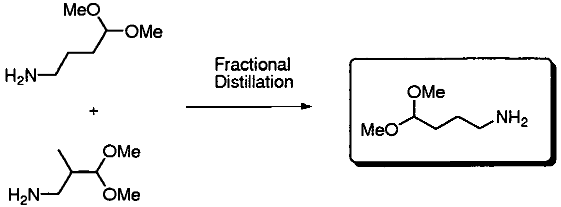

### Process development scale:

340 g of crude aminobutyraldehyde dimethyl acetal from the larger scale preparation was distilled in a 1 litre glass flask fitted with nitrogen bleed, 300 mm x 30 mm diameter stainless steel mesh fractionation column and reflux ratio head. The following fractions were collected:

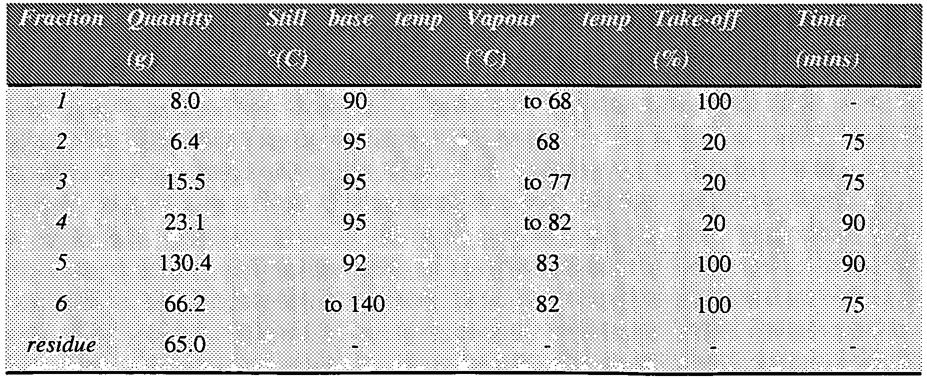

Analysis of these fractions by ¹H NMR and GC showed them to contain the following constituents:

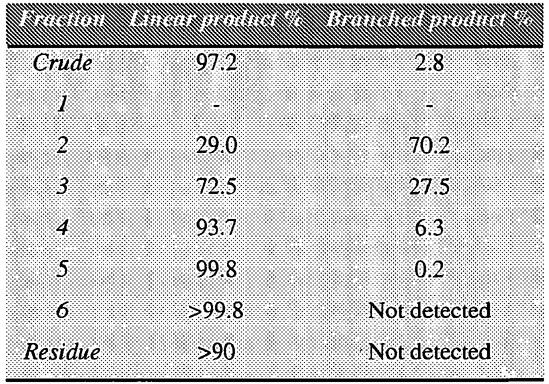

### 4-Aminobutyraldehyde dimethyl acetal

¹H NMR (CDCl₃, 400 MHz): δ 1.51 (2H, CH₂, m), 1.63 (2H, CH₂, m), 1.9 (2H, NH₂, bs), 2.71 (2H, C*H*₂N, t, *J*³ 7.1 Hz), 3.33 (6H, CH₃, s), 4.38 (1H, C*H*(OCH₃)₂, t, *J*³ 5.5 Hz).
¹³C{¹H} NMR (CDCl₃, 100 MHz): δ 28.9, 30.2, 42.1, 53.1, 104.8.

### 3-Amino-2-methylproprylaldehyde dimethyl acetal

¹H NMR (CDCl₃, 400 MHz); δ 0.94 (3H, CH₃, d, ³*J* 6.6 Hz), 1.16 (1H, NH₂, bs), 1.83 (1H, CH, m), 2.57 (1H, C*H*₂N, m), 2.79 (1H, C*H*₂N, m), 3.36 (3H, CH₃, s), 3.38 (3H, CH₃, s), 4.15 (1H, C*H*(OCH₃)₂, d, ³*J* 6.6 Hz).
¹³C{¹H} NMR (CDCl₃, 100 MHz): δ 13.4, 37.0, 39.1, 53.9, 54.9, 108.4.

GC Conditions: ZB5 column, 30 m x 0.32 mm x 1.0 µm, MeCN for sample preparation, 160 °C for 6 min, 4 min (branched dimethyl acetal), 5 min (linear dimethyl acetal).

## Claims

1. A method comprising
(a) providing an *N*-alkenylphthalimide;
(b) converting the alkenyl group to aldehyde functionality by hydroformylation;
(c) reacting the aldehyde with an alcohol or alkyl diol to form acetal or cyclic acetal functionality; and
(d) then forming a primary amine by deprotection of the amine.
wherein the hydroformylation step occurs in the presence of syngas and a catalyst which is a complex of rhodium with a bidentate phosphorous containing ligand.

2. The method of claim 1 further comprising fractional distillation after formation of the primary amine.

3. The method of claim 1 or 2 wherein the *N*-alkenylphthalimide is *N*-allylphthalimide.

4. The method of any one of claims 1-3 wherein the step of providing an N-alkenylphthalimide comprises reacting potassium phthalimide with an allyl halide.

5. The method of claim 4 wherein the allyl halide is allyl chloride or allyl bromide.

6. The method of any one of claims 1-5 wherein the resulting aldehyde regioisomers from the hydroformylation step are present in ratios of linear to branched of at least 2:1.

7. The method of claim 6 wherein the molar ratio of linear to branched aldehyde is at least 10:1, and wherein the alkenyl group is allyl.

8. The method of any one of claims 6 to 7 wherein the ligand is:

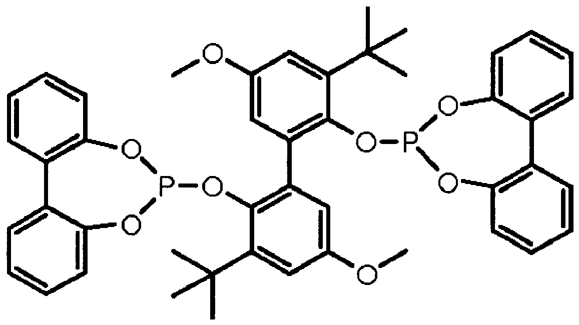

9. The method of any one of claims 1-8 wherein the alcohol in step (c) is methanol or ethanol.

10. A method of claim 9 wherein the alcohol is methanol.

11. The method of any one of claims 1-10 wherein the deprotection occurs via addition of a deprotecting reagent selected from an aqueous base, an amine or hydrazine,

12. A method of claim 11 wherein the deprotecting reagent is methylamine.

## Patentansprüche

1. Verfahren, das Folgendes umfasst:
(a) Bereitstellen eines N-Alkenylphthalimids;
(b) Umwandeln der Alkenylgruppe zu einer Aldehydfunktionalität durch Hydroformylierung;
(c) Umsetzen des Aldehyds mit einem Alkohol oder Alkyldiol zur Bildung einer Acetal- oder einer zyklischen Acetal-Funktionalität; und
(d) anschließende Bildung eines primären Amins durch Entschützung des Amins,
wobei der Hydroformylierungsschritt in Gegenwart von Synthesegas und einem Katalysator erfolgt, der aus einem Komplex von Rhodium mit einem Phosphor enthaltenden Bidentatliganden besteht.

2. Verfahren nach Anspruch 1, das ferner eine fraktionierte Destillation nach der Bildung des primären Amins umfasst.

3. Verfahren nach Anspruch 1 oder 2, wobei das N-Alkenylphthalimid ein *N*-Allylphthalimid ist.

4. Verfahren nach einem der Ansprüche 1-3, wobei der Schritt des Bereitstellens eines *N*-Alkenylphthalimids das Umsetzen von Kaliumphthalimid mit einem Allylhalogenid umfasst.

5. Verfahren nach Anspruch 4, wobei das Allylhalogenid Allylchlorid oder Allylbromid ist.

6. Verfahren nach einem der Ansprüche 1-5, wobei die sich aus dem Hydroformylierungsschritt ergebenden Aldehyd-Regioisomere in einem Verhältnis von geradkettig zu verzweigtkettig von mindestens 2:1 vorhanden sind.

7. Verfahren nach Anspruch 6, wobei das molare Verhältnis von geradkettigem zu verzweigtkettigem Aldehyd mindestens 10:1 beträgt und die Alkenylgruppe Allyl ist.

8. Verfahren nach einem der Ansprüche 6 bis 7, wobei der Ligand: 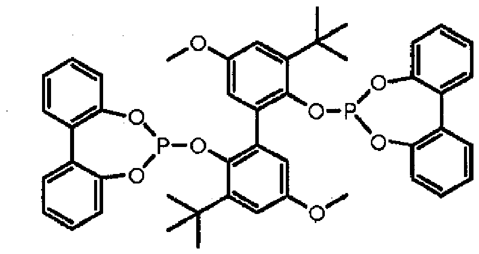 ist.

9. Verfahren nach einem der Ansprüche 1-8, wobei der Alkohol in Schritt (c) Methanol oder Ethanol ist.

10. Verfahren nach Anspruch 9, wobei der Alkohol Methanol ist.

11. Verfahren nach einem der Ansprüche 1-10, wobei die Entschützung über die Addition eines Entschützungsreagenzes geschieht, das ausgewählt ist aus einer wässrigen Base, einem Amin oder einem Hydrazin.

12. Verfahren nach Anspruch 11, wobei das Entschützungsreagenz Methylamin ist.

## Revendications

1. Procédé comprenant
(a) la fourniture d'un *N*-alcénylphtalimide ;
(b) la conversion du groupe alcényle en fonctionnalité aldéhyde par hydroformylation ;
(c) la réaction de l'aldéhyde avec un alcool ou un alkyldiol pour former une fonctionnalité acétal ou acétal cyclique ; et
(d) la formation ultérieure d'une amine primaire par déprotection de l'amine,
dans lequel l'étape d'hydroformylation est réalisée en présence d'un gaz de synthèse et d'un catalyseur qui est un complexe de rhodium avec un ligand contenant un phosphore bidenté.

2. Procédé selon la revendication 1, comprenant en outre une distillation fractionnelle après la formation de l'amine primaire.

3. Procédé selon la revendication 1 ou 2, dans lequel le N-alcénylphtalimide est le N-allylphtalimide.

4. Procédé selon l'une quelconque des revendications 1 à 3, dans lequel l'étape de fourniture d'un *N-*alcénylphtalimide comprend la réaction de phtalimide de potassium avec un halogénure d'allyle.

5. Procédé selon la revendication 4, dans lequel l'halogénure d'allyle est un chlorure d'allyle ou un bromure d'allyle.

6. Procédé selon l'une quelconque des revendications 1 à 5, dans lequel les régioisomères aldéhydiques résultant de l'étape hydroformylation sont présents dans des rapports de l'aldéhyde linéaire à l'aldéhyde ramifié d'au moins 2:1.

7. Procédé selon la revendication 6, dans lequel le rapport molaire de l'aldéhyde linéaire à l'aldéhyde ramifié est d'au moins 10:1, et dans lequel le groupe alcényle est un groupe allyle.

8. Procédé selon l'une quelconque des revendications 6 à 7, dans lequel le ligand est :

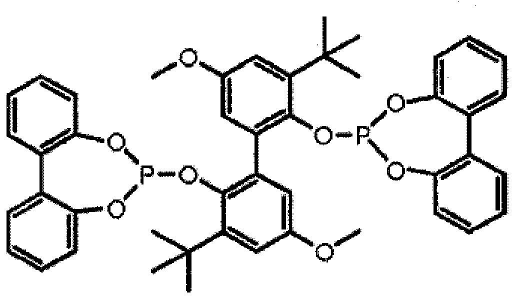

9. Procédé selon l'une quelconque des revendications 1 à 8, dans lequel l'alcool dans l'étape (c) est le méthanol ou l'éthanol.

10. Procédé selon la revendication 9, dans lequel l'alcool est le méthanol.

11. Procédé selon l'une quelconque des revendications 1 à 10, dans lequel la déprotection se produit par le biais de l'ajout d'un réactif de déprotection choisi parmi une base aqueuse, une amine ou une hydrazine.

12. Procédé selon la revendication 11, dans lequel le réactif de déprotection est la méthylamine.
